# EUROPEAN PATENT APPLICATION

(11) **EP 2 524 910 A1**
(43) Date of publication of application: **21.11.2012**
(21) Application number: 11166393.6
(22) Date of filing: 17.05.2011
(51) Int. Cl.: C07C 231/20, C07C 237/06

(54) **Process for the resolution of aminobutyramide**

(71) Applicant: DSM IP Assets B.V., 6411 TE Heerlen (NL)
(72) Inventor: BOESTEN, Wilhelmus Hubertus Joseph, 6132 BJ, SITTARD (NL); BOONEN, Jozef Johannes Catherina Jacobus, 5971 MA, Grubbenvorst (NL); WOESTENBORGHS, Pierre Louis, 3650, DILSEN (BE)
(74) Representative: De Vroom, Erik

(57) **Abstract**

The present invention relates to a process for the resolution of Schiff bases of racemic (*R,S*)-2-aminobutyramide to give optically pure (*S*)-2-aminobutyramide, an intermediate in the synthesis of levetiracetam.

## Description

### Field of the invention

The present invention relates to a process for the resolution of Schiff bases of racemic (*R,S*)-2-aminobutyramide to give optically pure (*S*)-2-aminobutyramide, an intermediate in the synthesis of levetiracetam.

### Background of the invention

Levetiracetam ((α*S*)-α-ethyl-2-oxo-1-pyrrolidineacetamide) is an anticonvulsant used to treat epilepsy. It is the *S*-enantiomer of etiracetam, structurally similar to the prototypical drug piracetam. Along with other anticonvulsants like gabapentin, it is also sometimes used to treat neuropathic pain. It is also used in veterinary medicine for similar purposes. Levetiracetam was first disclosed in EP 162036 indicating particular therapeutic properties distinguishing it from the racemic form.

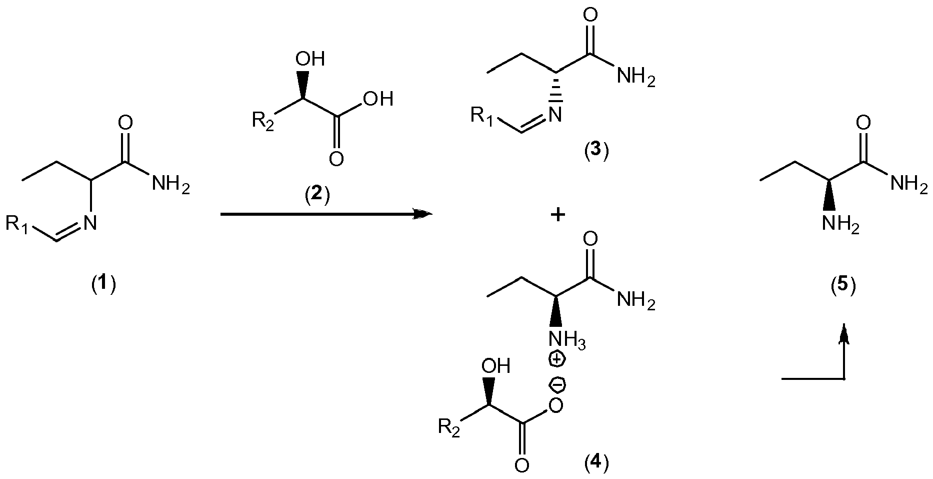

Several processes for obtaining levetiracetam have been disclosed. One promising approach is the reaction of (*S*)-2-aminobutyramide (**5**) with an alkyl 4-halobutyrate or with a 4-halobutyryl halide followed by cyclization as outlined in EP 162036. Clearly, said (*S*)-2-aminobutyramide (**5**) is a key intermediate in the preparation of levetiracetam and given the importance of the correct stereochemistry of levetiracetam also the correct stereochemistry in the key intermediates is of importance.

The separation of stereoisomers is considered to be one of the difficult tasks in chemistry since chiral compounds exhibit identical physical properties in non-chiral environments. Although several approaches for the preparation of optically pure (*S*)-2-aminobutyra*mide (**5**) have been reported, many of these are related to resolution of racemic* (*R,S*)-2-aminobutyramide (*e.g.* WO 2006/103696), optionally using catalytic amounts of an aldehyde such as described in JP 2007/191470. However, an approach directly starting from the Schiff base of racemic (*R,S*)-2-aminobutyramide (*i.e.* compound (**1**)) is unavailable whereas there is a need for this as said Schiff bases are highly suitable from a preparative point of view as these compounds may be conveniently isolated from the aqueous media that they are usually prepared in. This is in contrast with the parent 2-aminobutyramide which is highly soluble in water and consequently difficult to obtain in sufficient purity.

### Detailed description of the invention

In a first embodiment the present invention provides a process for the preparation of (*S*)-2-aminobutyramide (**5**) or a salt thereof comprising the steps of:
(a) Mixing a solvent and a compound of general formula (**1**) with an amine resolving acid of general formula (**2**) to obtain a salt of general formula (**4**);
(b) Treating the salt obtained in step (a) with an acid;
(c) Isolating (*S*)-2-aminobutyramide or a salt thereof from the mixture obtained in step (b)
   wherein R₁ is phenyl or substituted phenyl and R₂ is phenyl or substituted phenyl or -CH₂CO₂H or -CH(OH)CO₂H.

Resolution of racemic compounds is possible with optically pure reagents forming pairs of diastereomers which can be separated by conventional techniques in physical chemistry. Derivatization is possible by salt formation between an amine and a carboxylic acid. Simple de-protonation subsequently affords the pure enantiomer. Suitable carboxylic acids in this respect are amine resolving acids. An amine resolving acid is an optically active acid such as those commonly used for resolving amines by diastereoisomeric salt crystallization, (*e.g*. J. Jacques et al., "Enantiomers, Racemates, and Resolutions", Wiley, 1981; A. N. Collins et al., "Chirality in Industry", John Wiley & Sons, 1992). Examples of amine resolving acids and suitable in the context of the present invention are one of the enantiomers of malic acid, mandelic acid, tartaric acid and derivatives thereof such as di-O,O'-anisoyltartaric acid, di-O,O'-benzoyltartaric acid and di-O,O'-toluoyltartaric acid. Preferably the amine resolving acid is D-mandelic acid, ((**2**) with R₂ is phenyl) or L-tartaric acid, ((**2**) with R₂ is -CH(OH)CO₂H).

Preferred examples of racemic Schiff bases (**1**) are those wherein R₁ is phenyl or a substituted phenyl such as 3,5-dichloro-2-hydroxyphenyl, hydroxyphenyl and 4-nitrophenyl which compounds are prepared by reacting 2-aminobutyramide with 3,5-dichlorosalicylaldehyde, salicylaldehyde and 4-nitrobenzaldehyde respectively. The Schiff base (1) wherein R₁ is phenyl can be prepared by reacting 2-aminobutyramide with benzaldehyde.

The process of the present invention can be carried out in a variety of solvents. Alcohols are preferred as solvent. Most preferred solvents are ethanol, methanol, 4-methyl-2-pentanol, sec-butanol, n-butanol, n-propanol and isopropanol. Methanol was found to give excellent yields and e.e.-values when used in a process with tartaric acid as resolving agent which is advantageous because of the fact that methanol is readily available and relatively inexpensive.

Step (a) as outlined above results in the formation of a mixture of the salt of the amine resolving acid with (*S*)-2-aminobutyramide (*i.e*. compound (**4**)) and the (*R*)-form of compound (**1**) (*i.e*. compound (**3**)). Prior to liberating (*S*)-2-aminobutyramide (**5**) from the salt (**4**), the latter is optionally isolated, for example by means of filtration or centrifugation. The salt (**4**) thus obtained may optionally be further purified by washing with a solvent, re-crystallization or both. Solid salt (**4**) thus obtained is converted to (*S*)-2-aminobutyramide (**5**) by contacting with an acid. Suitable acids in this respect are inorganic acids such as hydrobromic acid, hydrochloric acid, nitric acid, sulfuric acid and the like. Finally, (*S*)-2-aminobutyramide (**5**) may be isolated by techniques such as centrifugation, filtration or sedimentation and the like. Preferably, (*S*)-2-aminobutyramide (**5**) is isolated as a salt such as the hydrobromic acid salt, the hydrochloric acid salt , the nitric acid salt , the sulfuric acid or other salts common to the skilled person. The (*S*)-2-aminobutyramide (**5**) thus obtained may optionally be further purified by washing with a solvent, re-crystallization or both.

In a second embodiment, (*S*)-2-aminobutyramide (**5**) or salt thereof is further converted into levetiracetam by reaction with alkyl 4-halobutyrate or with a 4-halobutyryl halide followed by cyclization and isolation of the product. Levetiracetam thus obtained may optionally be further purified by washing with a solvent, re-crystallization or both.

In a third embodiment, the present invention provides recovery of the undesired isomer. Thus, undesired (*R*)-isomer of 2-(benzylideneamino)butyramide is racemized and the racemic 2-(benzylideneamino)butyramide thus obtained is re-used in the process of the present invention. Hence, following step (a), the compound of general formula (**3**) wherein R₁ is as outlined above is treated with a base to form racemic 2-(benzylideneamino)butyramide.

### METHODS

### HPLC Analysis of (RS)-2-aminobutyric acid and (RS)-2-aminobutyramide

### HPLC conditions:

| | |
|---|---|
| Column: | Macherey-Nagel ET250/4 Nucleosil 120-5 C18 |
| Flow: | 1.2 ml/min |
| Column temperature: | 40°C |
| Injection volume: | 10 µl |

### Post-column derivatization:

| | |
|---|---|
| Flow OPA reagens: | 1 ml/min |
| λₑₓₜ: | 360 nm |
| λₑₘ: | 420 nm |
| Temperature reaction: | 40 °C |

### Eluens:

Dilute 1 M NaOH (20 mL) with approximately 1500 ml Milli-Q water and bring the pH to 3.0 with 1 M H₃PO₄. Dissolve 1-octanesulphonic acid monohydrate (234 mg; Acros art. 20.614.50) in 200 mL MeOH and 200 mL Milli-Q water and add this mixture to the NaH₂PO₄ solution and correct the pH value, if needed, to 3.0. Bring the total volume to 2.00 L using Milli-Q water.

### OPA reagens:

Dissolve 37 gram di-sodiumtetraborate decahydrate and 3.5 gram NaOH in water and fill up to 1 liter with Milli-Q water.

Dissolve 0.8 gram o-phtalic aldehyde in 10 ml absolute ethanol, add 1 ml mercaptoethanol and add this mixture to the borate buffer solution. Mix well.

The mixture has a limited storage life (max. 5 days)

| **Component** | **Retention Time (min)** |
|---|---|
| (*RS)*-2-aminobutyric acid | 3.2 |
| (*RS*)-2-aminobutyramide | 3.6 |
| Ammonia | 4.7 |

### Analysis method for the determination of the enantiomeric purity of 2-aminobutyramide.HCl

The presence of (*R*)-2-aminobutyric acid and NH₃ will lead to inaccurate results, due to the fact that (*R*)-2-aminobutyric acid is overlapping with (*R*)-2-aminobutyramide and NH₃ has the same retention time as (*S*)-2-aminobutyramide.

### HPLC conditions:

| | |
|---|---|
| Column: | Chrompack Cr(+) (150 x 4.0 mm ID) - Daicel Industries |
| Flow: | 0.8 ml/min |
| Column temperature: | 0 °C |
| Injection volume: | 10 µl |

### Post-column derivatization:

| | |
|---|---|
| Flow OPA reagens: | 1 ml/min |
| Temperature reaction: | 40 °C |

### Detection (Fluorescence detector (Waters 2475)):

| | |
|---|---|
| λₑₓₜ: | 360 nm |
| λₑₘ: | 420 nm |

### Eluens:

100 mM perchloric acid in water (pH ~ 1.0)

### OPA reagens:

Dissolve 37 gram di-sodiumtetraborate decahydrate and 3.5 gram NaOH in water and fill up to 1 liter with Milli-Q water.

Dissolve 0.8 gram o-phtalic aldehyde in 10 ml absolute ethanol, add 1 ml mercaptoethanol and add this mixture to the borate buffer solution. Mix well.

The mixture has a limited storage life (max. 5 days)

### EXAMPLES

### Example 1

### Preparation of 2-(benzylideneamino)butyramide ((1), R₁ = phenyl)

A reactor was charged with 9.181 kg 25% ammonia at a temperature of 25°C. Subsequently, sodium cyanide (1.069 kg) was added and the suspension was stirred until all solids were dissolved. Next, acetic acid (1.374 kg) was added within 1 hour while keeping the temperature between 25-30°C by cooling. Subsequently, propanal (97%, 1.242 kg) was added within 1 hour while keeping the temperature between 30-35°C and the mixture was stirred for 2.5 hours at 35°C and then cooled to 30°C. To the mixture acetone (1.504 kg) was added at a temperature of 25-30°C followed by an aqueous sodium hydroxide solution (50%, w/w, 0.286 kg) while keeping the temperature at 35°C at which temperature the mixture was stirred for 1 hour. Next, acetic acid (0.247 kg) was added followed by water (7.168 kg). The pressure in the reactor was decreased to 50 mbar and acetone was distilled off at a temperature not higher then 40°C until the acetone content was ≤2% w/w. Next, benzaldehyde (1.828 kg) was added within 4 hours while keeping the temperature between 30-35°C and the mixture was stirred for 1 hour at 35°C. The crystals formed were isolated by filtration and the crystal cake was washed with water (one part of water per part of crystal cake volume). The title product thus obtained was dried at 60°C and 30-50 mbar until the water content was below 1% w/w to yield 3.250 kg product with a chemical purity (determined by HPLC analysis) of 93 %. The yield based on propanal was 77%.

### Example 2

### Resolution of 2-(benzylideneamino)butyramide ((1), R₁ = phenyl) with D-mandelic acid in 4-methyl-2-pentanol

A reactor was charged with 4-methyl-2-pentanol (12.295 kg) and water (0.240 kg) after which racemic 2-(benzylideneamino)butyramide ((**1**), R₁ = phenyl, 5.719 kg) as obtained in Example 1 was added. The mixture was heated to 65°C until all solids were dissolved. Next, a solution of (R)-2-hydroxy-2-phenylacetic acid (D-mandelic acid, (**2**) with R₂ is phenyl, 2.219 kg) in 4-methyl-2-pentanol (7.868 kg) was added at 65°C within 30 minutes. The resulting solution was cooled slowly to 50°C and at about 53°C crystallization started. The mixture was stirred at 50°C for 1 hour, was cooled to 40°C and stirred for an additional 2 hours. The reaction mixture was filtered at 40°C and the crystal cake comprising of the D-mandelic acid salt of (*S*)-2-aminobutyramide was washed with 4-methyl-2-pentanol (4.225 kg) to yield wet D-mandelic acid salt of (*S*)-2-aminobutyramide (2.350 kg). The chemical purity (on dry basis) is ≥99% with an e.e. of ≥98.5% (HPLC). The filtrate was used for racemisation of the unwanted isomer and recovery of racemic 2-aminobutyramide (Example 4).

### Example 3

### Resolution of 2-(benzylideneamino)butyramide ((1), R₁ = phenyl) with D-mandelic acid in 1-butanol

Racemic 2-(benzylideneamino)butyramide ((**1**), R₁ = phenyl, 100.0 g, from Example 1) was dissolved in a mixture of 1-butanol (180.0 g) and water (5.0 g) at 60°C. The mixture was heated to 65°C. Next, a solution of (*R*)-2-hydroxy-2-phenylacetic acid (D-mandelic acid, (**2**) with R₂ is phenyl, 39.0 g) in 1-butanol (100 g) was added at 65°C within 30 minutes. The mixture was cooled to 40°C in approximately 2 hours after which crystals of the D-mandelic acid salt of (*S*)-2-aminobutyramide were isolated by filtration and washed with n-butanol (25 mL). After drying the yield of the D-mandelic acid salt of (*S*)-2-aminobutyramide was 30.0 g (e.e. = 99.2%)

### Example 4

### (S)-2-Aminobutyramide hydrochloric acid salt ((5), HCl salt) from the D-mandelic acid salt of (S)-2-aminobutyramide

A reactor was charged with acetone (8.220 kg) and wet D-mandelic acid salt of (*S*)-2-aminobutyramide (2.350 kg, contains ~10% w/w of 4-methyl-2-pentanol) as obtained in Example 2. In 1 hour an aqueous solution of hydrochloric acid (37%, 0.997 kg) was added while the temperature was kept below 30°C. The mixture was stirred for 3 hours at 30°C, cooled to a temperature below 25°C and the crystals of the title product were isolated by filtration, washed with acetone (2.108 kg) and dried at 60°C and 50 mbar to yield to title product (1.110 kg) with a chemical purity: ≥99% and an e.e. of ≥99.5%.

### Example 5

### Racemisation and recovery of 2-aminobutyramide from (R)-2- (benzylideneamino)butyramide ((3), R₁ = phenyl)

The organic phase from the filtrate obtained in Example 2 (31.16 kg) was heated to 50°C after which an aqueous solution of sodium hydroxide (50%, 0.623 kg) was added and the resulting mixture was stirred for 3 hours at 50°C. Water (12.92 kg) was added and sulphuric acid (96%, approximately 1.22 kg) was added until the pH reached a value of 2.8±0.3 after which the phases were separated. The aqueous phase was used in the synthesis of 2-(benzylideneamino)butyramide ((**1**), R₁ = phenyl) by adding it to the reaction mixture in a preparation similar to the one described in Example 1 just before the addition of benzaldehyde.

### Example 6

### Resolution of 2-(benzylideneamino)butyramide ((1), R₁ = phenyl) with L-tartaric acid

Racemic 2-(benzylideneamino)butyramide ((**1**), R₁ = phenyl, 30.0 g) was dissolved in a mixture of methanol (90.0 g) and water (3.0 g) at 40°C. The mixture was heated to 50°C and (2*R*,3*R*)-2,3-dihydroxybutanedioic acid (L-tartaric acid, (**2**) with R₂ is -CH(OH)CO₂H, 11.5 g) was added. The mixture was cooled to 35°C in approximately 2 hours after which crystals of the L-tartaric acid salt of (*S*)-2-aminobutyramide were isolated by filtration and washed with methanol (25 mL). After drying the yield of the L-tartaric acid salt of (*S*)-2-aminobutyramide was 12.9 g (e.e. = 95%)

### Example 7

### Resolution of 2-(4-hydroxybenzylideneamino)butyramide ((1), R₁ = 4-hydroxyphenyl) with D-mandelic acid in 4-methyl-2-pentanol

Racemic 2-(4-hydroxy-benzylideneamino)butyramide ((**1**), R₁ =4-hydroxyphenyl, 5.0 g) as obtained in Example 1 was dissolved in a mixture of 4-methyl-2-pentanol (20.0 g) and water (0.40 g) at 60°C. The mixture was heated to 65°C. Next, (*R*)-2-hydroxy-2-phenylacetic acid (D-mandelic acid, (**2**) with R₂ is phenyl, 3.65 g) was added at 65°C. The mixture was cooled to 20°C overnight after which crystals of the D-mandelic acid salt of (*S*)-2-aminobutyramide were isolated by filtration and washed with MIBC (10 mL). After drying the yield of the D-mandelic acid salt of (*S*)-2-aminobutyramide was 2.30 g (e.e. = 77%).

## Claims

1. A process for the preparation of (*S*)-2-aminobutyramide or a salt thereof comprising the steps of:
(a) Mixing a solvent and a compound of general formula (1) 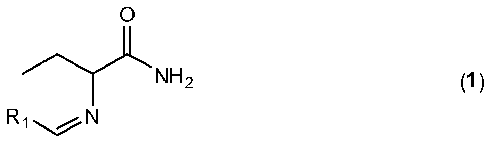 with an amine resolving acid of general formula (2) 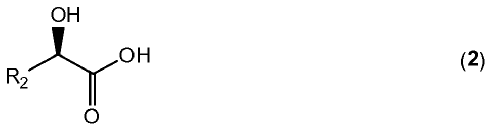 to obtain a salt of general formula (4); 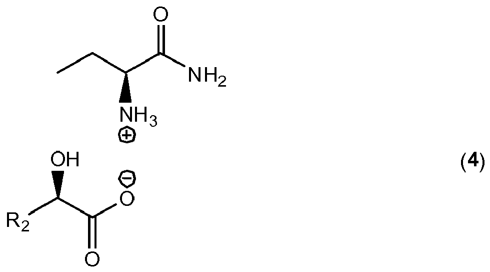
(b) Treating the salt obtained in step (a) with an acid;
(c) Isolating said (*S*)-2-aminobutyramide or a salt thereof from the mixture obtained in step (b)
wherein R₁ is phenyl or substituted phenyl and R₂ is phenyl or substituted phenyl or -CH₂CO₂H or -CH(OH)CO₂H.

2. Process according to claim 1 wherein said solvent is an alcohol.

3. Process according to any one of claims 1 to 2 wherein said amine resolving acid of general formula (**2**) is D-mandelic acid or L-tartaric acid.

4. Process according to any one of claims 1 to 3 wherein a compound of general formula (**3**) 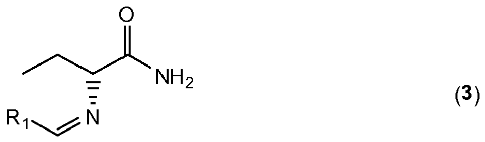 present in the mixture as obtained after step (a) is treated with base to form said compound of general formula (**1**).
